# EUROPEAN PATENT APPLICATION

(11) **EP 3 213 673 A1**
(43) Date of publication of application: **06.09.2017**
(21) Application number: 17158008.7
(22) Date of filing: 24.02.2017
(51) Int. Cl.: A61B 5/00, A61B 5/0205

(54) **SMART SPORTS EYEWEAR**

(30) Priority: 01.03.2016 CN 201610115476; 28.04.2016 US 201615140805
(71) Applicant: Shanghai Xiaoyi Technology Co., Ltd., Pudong New Area Shanghai 201203 (CN)
(72) Inventor: LIU, Yi, Free Trade Zone, Pudong New Area, Shanghai 201203 (CN)
(74) Representative: Finnegan Europe LLP

(57) **Abstract**

A wearable device (110) includes a display component (210) configured to display a virtual image. The wearable device (110) also includes a first sensor (250) configured to generate a first signal indicative of a physiological condition of a user. The wearable device (110) further includes a controller (220) configured to determine the physiological condition based on the first signal, and control the display component (210) to display the physiological condition.

## Description

### CROSS-REFERENCE TO RELATED APPLICATIONS

This application is based upon and claims priority to Chinese Patent Application No. 201610115476.9, filed March 1, 2016, and United States Patent Application No. 15/140,805, filed April 28, 2016, the entire contents of which are incorporated herein by reference.

### TECHNICAL FIELD

The present disclosure relates generally to the field of wearable devices, and more particularly, to a smart sports eyewear having extendable functions.

### BACKGROUND

To lead a healthy and active lifestyle, more and more people are engaging in various sports and fitness activities during any available time and in any outdoor or indoor environments. However, people usually do not have convenient or effective ways to form an overall and accurate understanding about their physiological reactions during exercises. Therefore, adverse events may happen due to over exercising. These adverse events, without timely treatment, may cause serious harm to the health.

Moreover, when people are exercising in an unfamiliar environment or a complex terrain, they may easily get lost. However, it is not convenient to carry the conventional navigation devices during exercises.

The disclosed system and method address one or more of the problems discussed above.

### SUMMARY

Consistent with one disclosed embodiment of the present disclosure, a wearable device is provided. The wearable device may include a display component configured to display a virtual image. The wearable device may also include a first sensor configured to generate a first signal indicative of a physiological condition of a user. The wearable device may further include a controller configured to: determine the physiological condition based on the first signal; and control the display component to display the physiological condition.

Consistent with another disclosed embodiment of the present disclosure, a method is provided. The method may include generating a first signal indicative of a physiological condition of a user. The method may also include determining the physiological condition based on the first signal. The method may also include displaying the physiological condition on a display panel. The method may further include generating a virtual image of the display panel.

The method may further comprise: determining whether the physiological condition is within a predetermined range; and if the physiological condition is not within the predetermined range, displaying a warning message on the virtual image.

The method may further comprise: generating an advice based on the physiological condition; and displaying the advice on the virtual image.

The method may further comprise overlaying the virtual image on a physical environment surrounding the user.

The method may further comprise: generating a second signal indicative of a movement of the user; and determining the movement of the user based on the second signal.

The method may further comprise: generating a third signal indicative of a location of the user; and determining the location of the user based on the third signal.

The method may further comprise: generating images of a physical environment surrounding the user; and determining a movement of the user based on the images of the physical environment.

The method may further comprise: establishing a communication with a third-party device; determining whether an emergency has occurred; and if the emergency has occurred, sending a rescue request to the third-party device.

The present disclosure also includes a processor-readable medium comprising instructions which, when executed by a processor, cause the processor to perform the method as disclosed herein. The processor may implement the controller of the wearable device disclosed herein.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this specification, illustrate embodiments consistent with the present disclosure and, together with the description, serve to explain the principles of the present disclosure.
Fig. 1 is a schematic diagram illustrating a system, according to an exemplary embodiment.
Fig. 2 is a schematic diagram illustrating a smart eyewear used in the system illustrated in Fig. 1, according to an exemplary embodiment.
Fig. 3 is a block diagram of an exemplary smart eyewear, consistent with the smart eyewear illustrated in Fig. 2.
Fig. 4 is a schematic diagram illustrating an exemplary implementation of an augmented-reality display module in the smart eyewear shown in Fig. 3.
Fig. 5 is a flowchart of a method performed by the smart eyewear shown in Fig. 3, according to an exemplary embodiment.

### DETAILED DESCRIPTION

Reference will now be made in detail to exemplary embodiments, examples of which are illustrated in the accompanying drawings. The following description refers to the accompanying drawings in which the same numbers in different drawings represent the same or similar elements unless otherwise represented. The implementations set forth in the following description of exemplary embodiments do not represent all implementations consistent with the invention. Instead, they are merely examples of devices and methods consistent with aspects related to the invention as recited in the appended claims.

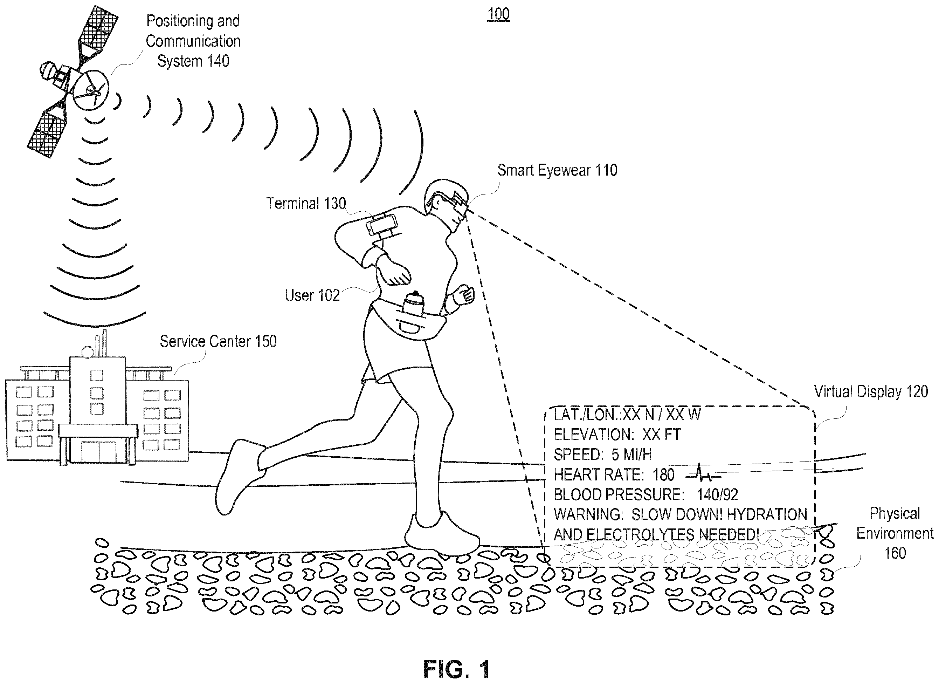

Fig. 1 is a schematic diagram illustrating a system 100, according to an exemplary embodiment. The system 100 may monitor and collect information regarding the physiological reactions of a user 102 during indoor or outdoor sports activities. The system 100 may also analyze the physiological reactions in real time and provide health and safety-related advice to user 102. The system 100 may further collect information regarding the surrounding physical environment and the position of user 102, and provide navigation for user 102. The system 100 may further enable the communication between user 102 and a third party, such as a rescue team or a doctor, so as to facilitate the search, rescue, and medical treatment of user 102 during an emergency. Referring to Fig. 1, system 100 may include a smart eyewear 110, a virtual display 120, a terminal 130, and a positioning and communication system 140.

Smart eyewear 110 may be implemented as a pair of smart glasses, a smart goggle, a head-mounted display, a smart helmet, etc. Smart eyewear 110 may include one or more wearable biosensors configured to measure various health indexes and physiological conditions of user 102 during exercises. Smart eyewear 110 may also include one or more positioning sensors and/or devices configured to detect the position and movement of user 102. Smart eyewear 110 may further include one or more imaging sensors and/or cameras configured to capture images of the physical environment 160 surrounding user 102. Smart eyewear 110 may further have computing power to process the above information.

Smart eyewear 110 may generate a virtual display 120 in user 102's field of view. Virtual display 120 may display a combination of graphics and texts to describe user 102's real-time physiological conditions, to provide exercise advices, to indicate user 102's location, etc. Referring to the example shown in Fig. 1, while user 102 is running, virtual display 120 may display user 102's speed, heart rate, blood pressure, and location information, such as the latitude, the longitude, and the elevation. Since the physiological condition suggests that user 102 probably has over exercised and is in a dehydrated state, virtual display 120 may further display a warning message alerting user 102 to slow down and take water and electrolytes.

Virtual display 120 does not need to be projected or displayed on a physical screen, and thus has several advantageous features. First, virtual display 120 can move together with user 102's field of view and thus can be conveniently viewed by user 102 despite the constant movement of user 102. Moreover, the size of virtual display 120 is not limited by the size of a screen. As described below, smart eyewear 110 may adjust the size of virtual display 120 as needed. For example, virtual display 120 may be configured to have a large size, which is easily viewed by user 120 during exercises. Further, without the need for a physical screen, smart eyewear 110 may have a light weight and may be suitable for being worn by user 102 during exercises.

Smart eyewear 110 may overlay virtual display 120 on physical environment 160 surrounding user 102, so as to provide a sense of augmented reality. This way, user 102 may simultaneously view virtue display 120 and at least part of physical environment 160, without refocusing the eyes. Therefore, virtual display 120 may present the physiological information and/or navigation information for user 102 without obstructing user 102's view of physical environment 160, and therefore may ensure the safety of user 102.

In some exemplary embodiments, the system 100 may include terminal 130 configured to collaborate with smart eyewear 110 to provide various functions. Terminal 130 may also be an electronic device wearable by user 102 during exercises. For example, terminal 130 may be a smart phone, a tablet computer, a smart watch, a smart bracelet, a smart camera, a personal digital assistant (PDA), a medical device, an ebook reader, etc.

Similar to smart eyewear 110, terminal 130 may be configured to perform various functions. For example, terminal 130 may include additional biosensors configured to detect physiological reactions of user 102 during exercise. Terminal 130 may also include high-resolution cameras configured to film physical environment 160. Terminal 130 can also conduct a telephone call with a third party device, store large volume of multimedia entertainment content, provide global position system (GPS) navigation, generate exercise advice based on user 102's physiological reactions, and the like.

Terminal 130 may form a binding relationship with smart eyewear 110 and communicate with smart eyewear 110 in a wired or wireless manner, such as through a connection cable or a Bluetooth link. Terminal 130 may transmits various information, such as the detected physiological reactions, the generated exercise advice, or the stored entertainment content, to smart eyewear 110 for displaying on virtual display 120. This way, terminal 130 may serve to extend and expand the functions of smart eyewear 110. Moreover, since terminal 130 may share the burden of collecting, storing, and/or processing data, smart eyewear 110 may have less requirement for the hardware, and thus can be made smaller and lighter, which is desirable for use in sports and exercises.

Positioning and communication system 140 may be used by smart eyewear 110 and/or terminal 130 to determine the location of user 102 and/or to provide communication service to user 102. For example, positioning and locating system 140 may be a satellite network formed by multiple satellites, or a cellular network formed by multiple wireless transceivers (for example, base stations). For example, smart eyewear 110 may receive signals from positioning and communication system 140 to determine the location of user 102 using triangulation or any other method known in the art. For another example, smart eyewear 110 may make a telephone call with a device in a third-party site, such as service center 150, through positioning and communication system 140.

Service center 150 may be located in a remote site and provide service to user 102 to ensure sports safety. For example, service center 150 may be a rescue center that can provide aid to user 102 when user 102 runs into an imminent danger. For another example, service center 150 may be a doctor's or physical therapist's office that can provide professional advice to user 102 when user 102 suffers an injury or adverse reaction during exercises. In exemplary embodiments, when detecting an adverse event, such as abnormal physiological reactions of user 102, smart eyewear 110 may automatically report the adverse event and user 102's location to service center 150. Smart eyewear 110 may also establish a telephone call with service center 150 so that user 102 may receive advice and guidance from service center 150 to properly react to the adverse event. Smart eyewear 110 may even establish a video conference so that the professionals at service center 150 can visually examine user 102's condition.

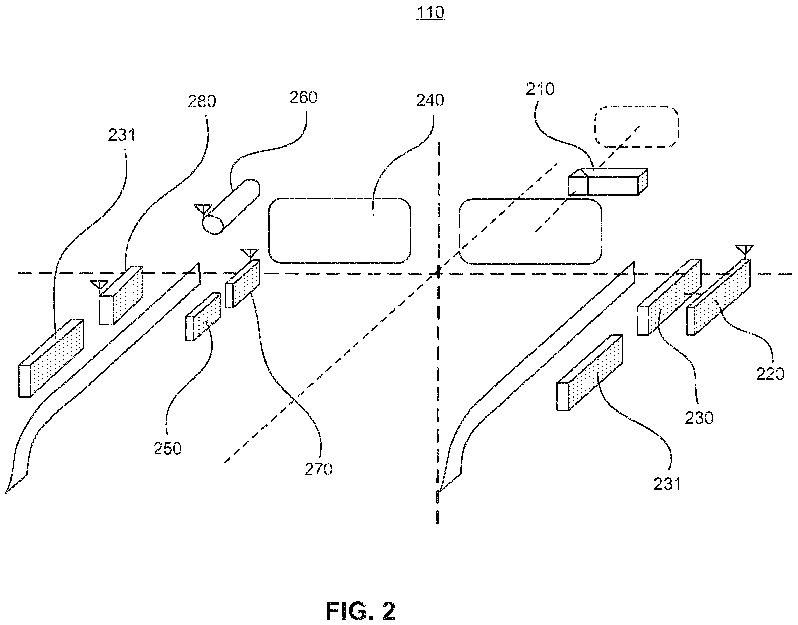

Fig. 2 is a schematic diagram illustrating a smart eyewear 110, according to an exemplary embodiment. For example, smart eyewear 110 may be a pair of smart glasses. Referring to Fig. 2, smart eyewear 110 may include one or more of the following components: an augmented-reality (AR) display module 210, a controller 220, a power component 230, one or more extended power sources 231, lens 240, a sensor module 250, an imaging module 260, a locating module 270, and a telecommunication module 280.

In the example illustrated in Fig. 2, smart eyewear 110 may be implemented as a pair of smart glasses wearable by user 102. However, it is contemplated that the technical solution provided by the present disclosure may be applied to any wearable device.

In the disclosed embodiments, smart eyewear 110 may be configured to be prescription glasses, magnifying glasses, non-prescription glasses, safety glasses, sunglasses, etc. Additionally, smart eyewear 110 may include parts of a frame and earpieces, nosepieces, etc., to prevent smart eyewear 110 from falling off from user 102 during exercises. Controller 220, power component 230, extended power source 231, sensor module 250, imaging module 260, locating module 270, and telecommunication module 280 may be attached, for example, to a temple or brow bar of the frame, so as not to block user 102's visual field. In contrast, the AR display module 210 and the lens 240 may be attached to an eyewire of the frame, such that user 102 can see virtual display 120 and/or physical environment 160 through AR display module 210 and lens 240.

AR display module 210 may include a micro-display and an associated optical assembly that are integrated in a small-sized box. The micro-display is placed in front of the user 102's eye(s). Controller 220 may control the micro-display to display images. The optical assembly may include one or more optical devices configured to generate a magnified virtual image of the image shown on the micro-display. Such virtual image, i.e., virtual display 120, can be viewed by user 102. Virtual display 120 may be overlaid on physical environment 160 to create an augmented reality. In some exemplary embodiments, smart eyewear 110 may include only one AR display module 210 placed in front of one eye of user 102 for monocular viewing. In some embodiments, smart eyewear 110 may include multiple AR display modules 210, with at least one AR display module 210 being placed in front of each eye for binocular viewing.

Controller 220 may include high-speed integrated circuitry configured to receive, process, and display various types of information. Controller 220 may establish wireless or wired communication with other components of smart eyewear 110 (for example, sensor module 250, imaging module 260, and locating module 270) and other devices (for example, terminal 130), and exchange data, signals, and commands with these components and/or devices. Controller 220 may filter, analyze, process, and store these data and signals, and generate exercise advice and navigation information for user 102.

Power component 230 may include one or more power sources, such as a lithium-ion battery array. In some embodiments, power component 230 may also include a power management system and any other components associated with the generation, management, and distribution of power in wearable eyewear 110.

Occasionally, user 102 may need to use wearable eyewear 110 uninterruptedly for an extended time or may have no easy access to a charging port during exercises. Thus, extended power source 231 may be used to provide extra power for smart eyewear 110. Extended power source 231 may be a lithium-ion battery pack. Smart eyewear 110 may include one or more slots/interfaces to allow easy installation and uninstallation of extended power source 231. For example, if user 102 will run an outdoor marathon or go to a hiking trip that lasts for a few days, user 102 may install one or more extended power sources 231 on smart eyewear 110. In contrast, if the exercise will only last for a short time, user 102 may remove all extended power sources 231 from smart eyewear 110 to reduce the weight of smart eyewear 110.

Lens 240 may be designed according to the specific need of user 102. Lens 240 may be a corrective lens if user 102 has certain vision deficiency. The corrective lens may be single vision, multifocal, or varifocal lens. Lens 240 may also be shatter-resistant plastic lenses to protect user 102's eyes from flying debris or dust. Lens 240 may also be a photochromic lens to protect user 102's eyes from bright light and ultraviolet light. Lens 240 may even comprise optical filters to enable user 102 to view three-dimensional images displayed by virtual display 120.

Sensor module 250 may include one or more biosensors configured to generate various signals quantitatively indicative of the physiological conditions of user 102, such as electrocardiography (ECG) signals indicative of cardiac activity, photoplethysmogram (PPG) signals indicative of changes in arterial blood volume remote from user 102's heart, galvanic skin response (GSR) signals indicative of electrical conductance of user 102's skin (i.e., the amount of sweat-induced moisture on the skin), bioimpedance signals indicative of hemodynamic characteristics within the brain, oximeter signals indicative of blood oxygen levels, sphygmomanometer signals indicative of arterial pressure, body temperature signals, heart rate signals, and/or any other signals indicative of a physiological condition of user 102. These biosensors may non-invasively obtain the respective signals. Each biosensor may include a detector configured to sample a physiological parameter, such as the concentration of a physiological substance, from a small area of surface skin. Each biosensor may further include a converter configured to convert the detected physiological parameter into an electronic signal that can be processed by controller 220.

Sensor module 250 may also include one or more sensors to provide status assessments of the movement of user 102, i.e., smart eyewear 110. In some exemplary emboduments, sensor module 250 may include one or more barometric sensors, proximity sensors, magnetometers, gyroscopes, accelerometers, motion detectors, depth sensors, etc. For example, sensor module 250 may include a proximity sensor configured to detect the presence of nearby objects without any physical contact. For another example, sensor module 250 may also include an inertial measurement unit (IMU) configured to measure a position, an orientation, an acceleration, a velocity, a heading, or an angular rate of smart eyewear 110. For example, the IMU may be a 6-degree of freedom (6 DOF) IMU. A 6 DOF IMU consists of a 3-axis accelerometer, 3-axis angular rate gyros, and sometimes a 2-axis inclinometer. The 3-axis angular rate gyros may provide signals indicative of the pitch rate, yaw rate, and roll rate of smart eyewear 110. The 3-axis accelerometer may provide signals indicative of the acceleration of smart eyewear 110 in the x, y, and z directions.

Imaging module 260 may include cameras and/or image sensors configured to detect and convert optical signals in the near-infrared, infrared, visible, and ultraviolet spectrums into electrical signals. The electrical signals may be used to form an image or a video stream (i.e. image data) based on the detected signal. The image data may be sent to controller 220 for further processing. For instance, controller 220 may display the image data on virtual display 120, or transmit the image data to service center 150. Examples of image sensors may include semiconductor charge-coupled devices (CCD), active pixel sensors in complementary metal-oxide-semiconductor (CMOS), or N-type metal-oxide-semiconductor (NMOS).

In one exemplary embodiment, imaging module 260 may include at least one outward-facing camera/image sensor to generate image data about physical environment 160. In another exemplary embodiment, image module 260 may include at least one user-facing eye tracking sensors configured to monitor and/or track a viewing direction of user 102 based on the position of one or both of user 102's eyes, and provide an output relating to the viewing direction of user 102 (for example, a direction of user 102's gaze).

Locating module 270 may include any device capable of providing a signal that indicates the location of smart eyewear 110, i.e., user 102. For example, locating module 270 could embody a global navigation satellite system (GNSS) receiver, such as a GPS device, that receives signals transmitted by a plurality of geosynchronous earth orbiting satellites in order to triangulate the location of smart eyewear 110. In some embodiments, locating module 270 may repeatedly forward a location signal (for example, a GPS signal) to an IMU to supplement the IMUs ability to compute position and velocity, thereby improving the accuracy of the IMU.

Telecommunication module 280 may be configured to establish a communication between user 102 and a third party, such as service center 150, through a satellite network or a cellular network. For example, when it is determined that user 102 suffers an injury or adverse event, telecommunication module 280 may automatically dial service center 150 to enable user 102 to speak to a member of service center 150. Telecommunication module 280 may also transmit user 102's physiological conditions and location information, and images of physical environment 160 to service center 150.

In exemplary embodiments, each of AR display module 210, controller 220, power component 230, extended power source 231, sensor module 250, imaging module 260, locating module 270, and telecommunication module 280 may be provided in individual modules that are water resistant, dust proof, and shock proof. Among the above components, extended power source 231, sensor module 250, imaging module 260, locating module 270, and telecommunication module 280 may be optional. Moreover, different sensor modules 250 may be used in smart eyewear 110 to detect different aspects of the physiological reaction and movement of user 102 during exercise. User 102 may select, according to the specific exercise needs, which optional component to be included in smart eyewear 110. Smart eyewear 110 may include slots, ports, and/or interfaces to receive each optional module and allow convenient installation and uninstallation of the optional components. For example, for indoor sports, user 102 may uninstall locating module 270 from smart eyewear 110 to reduce the weight of smart eyewear 110. For another example, if user 102 wants to closely monitor the heart rate during exercises, user 102 may install a sensor module 250 capable of measuring the heart rate.

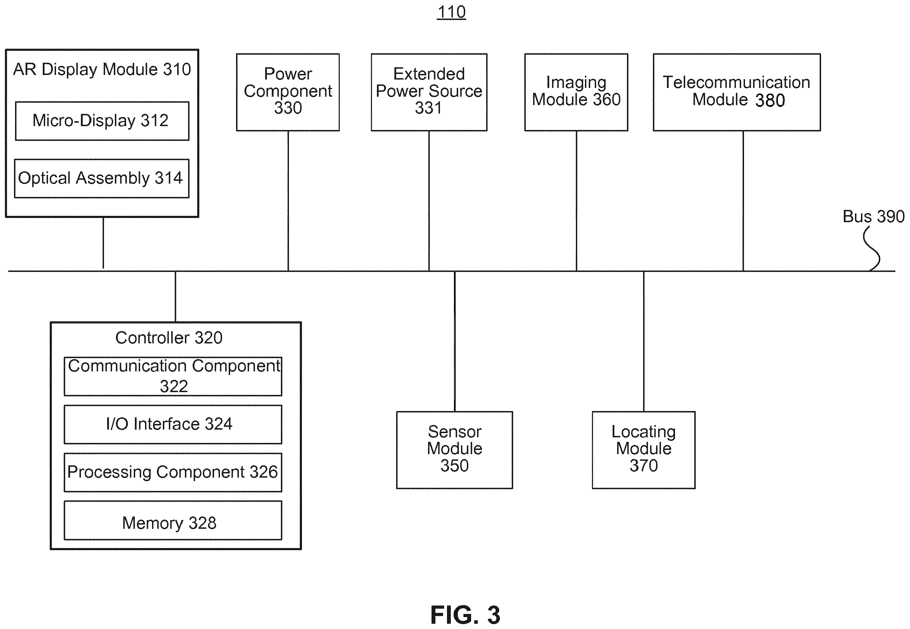

Fig. 3 is a block diagram of an exemplary smart eyewear 110, consistent with smart eyewear 110 depicted in Fig. 2. For example, smart eyewear 110 may be used in system 100. Referring to Fig. 3, smart eyewear 110 may include one or more of the following components: an AR display module 310, a controller 320, a power component 330, one or more extended power sources 331, a sensor module 350, an imaging module 360, a locating module 370, and a telecommunication module 380. The above components may be connected to each other via a bus 390. While a bus architecture is shown in Fig. 3, any suitable architecture may be used, including any combination of wired and/or wireless networks. Additionally, such networks may be integrated into any local area network, wide area network, the Internet, cellular network, radio network, and/or satellite network.

Controller 320 may include a communication component 322, an input/output (I/O) interface 324, a processing component 326, and a memory 328. One or more of the components of controller 320 may be implemented as one or more application-specific integrated circuits (ASICs), digital signal processors (DSPs), digital signal processing devices (DSPDs), programmable logic devices (PLDs), field programmable gate arrays (FPGAs), controllers, micro-controllers, microprocessors, or other electronic components, for performing functions consistent with controller 320. These components may be configured to transfer data and send or receive instructions between or among each other.

Communication component 322 may be configured to facilitate communication, wired or wirelessly, between controller 320 and other components of smart eyewear 110 or devices other than smart eyewear 110 (for example, terminal 130). Communication component 322 may access a wireless network based on one or more communication standards, such as Wi-Fi, LTE, 2G, 3G, 4G, 5G, etc. In one exemplary embodiment, communication component 322 may receive a broadcast signal or broadcast associated information from an external broadcast management system via a broadcast channel. In one exemplary embodiment, communication component 322 may further be configured to implement short-range communications based on a near field communication (NFC) technology, a radio frequency identification (RFID) technology, an infrared data association (IrDA) technology, an ultra-wideband (UWB) technology, a Bluetooth (BT) technology, or other technologies. For example, communication component 322 may exchange information with other components of smart eyewear 110 through a Bluetooth link.

I/O interface 324 may include one or more digital and/or analog devices configured to consolidate data/signals it received from communication component 322 and relay the data/signals to processing component 326. For example, I/O interface 324 may send the signals generated by sensor module 350 to processing component 326 for further processing. I/O interface 324 may also receive display signals from processing component 326, and send the display signals to AR module 310 for generating virtual display 120.

Processing component 326 may include any appropriate type of general purpose or special-purpose microprocessor, digital signal processor, central processing unit, circuitry, etc. Processing component 326 may be configured to receive and process the data generated by sensor module 350, imaging module 360, and locating module 370. Processing component 326 may also be configured to control the operation of AR display module 310, power component 330, and telecommunication module 380.

Processing component 326 may determine user 102's physiological conditions based on signals generated by sensor module 350 and further generate an advice based on the physiological conditions. For example, sensor 350 may be configured to detect the heart rate of user 102. When processing component 326 determines that the heart rate is outside a predetermined normal human heart rate range, processing component 326 may display a warning message on virtual display 120, such as a phrase in bold font or a flashing red-color sign. Processing component 326 may also generate an audible alarm and/or generate a vibration. Moreover, processing component 326 may display an exercise advice on virtual display 120, such as suggesting user 120 to lower the intensity of exercises, take a break, drink water, etc.

Processing component 326 may also determine user 102's movement based on signals generated by sensor module 350. For example, sensor module 350 may include an IMU. Processing component 326 may use the IMU signals to determine the position, forward velocity, angular velocities, and angular orientation (attitude) of user 102, i.e., smart eyewear 110. Processing component 326 may calculate forward velocity of smart eyewear 110 by integrating a signal indicative of forward acceleration from the IMU. Processing component 326 may also receive signals indicative of the angular rates (roll rate, yaw rate, and pitch rate) of smart eyewear 110 from the IMU. By integrating the angular rates, processing component 326 may determine the attitude or angular orientation (roll, heading, and pitch) of smart eyewear 110.

Processing component 326 may also determine user 102's position based on signals generated by locating module 370. For example, locating module 370 may be a GPS receiver. Processing component 326 may determine user 102's GPS coordinates and provide navigation for user 102 based on the GPS signals. Moreover, by combining the GPS signals with user 102's movement information, processing component 326 may accurately determine user 102's moving trajectory.

Processing component 326 may also execute various programs to process the image data generated by imaging module 360. The image data may include data associated with physical environment 160. For example, processing component 326 may improve user 102's vision by displaying on virtual display 120 the part of physical environment 160 that is outside user 102's field of view. For another example, by analyzing the change of physical environment 160, processing component 326 may determine the movement of user 102.

Based on the above-described signals regarding user 102 and physical environment 160, processing component 326 may further determine whether user 102 encounters an emergency. For example, if the detected physiological condition exceeds a predetermined range, processing component 326 may determine that user 102 experiences a medical emergency and needs help. For another example, based on the movement information of user 102 and images of physical environment 160, processing component 326 may determine that user 102 had an accident. If it is determined an emergency has occurred, processing component 326 may trigger telecommunication module 380 to send a rescue request to service center 150.

Processing component 326 may be configured to generate control signals used for controlling AR display module 310 to produce virtual display 120. In some exemplary embodiments, processing component 326 may perform various methods to optimize the image qualities, such as sharpness, color accuracy, brightness, or contrast ratio, of virtual display 120. For example, processing component 326 may optimize the brightness and contrast ratio of virtual display 120 based on one or more conditions, such as brightness, of physical environment 160 sensed by imaging module 360, so as to improve the user experience of the augmented reality. Particularly, when conditions of physical environment 160 is changing, such as changing from indoor to outdoor, processing component 326 may adjust brightness and contrast ratio of virtual display 120 accordingly.

Processing component 326 may also be configured to optimize the position of virtual display 120 in user 102's view of field. Based on the sensed physical environment 160, processing component 326 may render virtual display 120 in a position that does not impede viewing of real objects in physical environment 160. Moreover, processing component 326 may track the changes of user 102's head orientation and gaze direction, and/or physical environment 160, and constantly reposition virtual display 120.

Memory 328 may be any type of computer-readable medium, such as flash memory, random access memory, or firmware, configured to store data and/or instructions to support the operation of smart eyewear 110. Memory 328 may store the data received from other components of smart eyewear 110 and/or terminal 130. Memory 328 may also store instructions used by the processing component 326 to process the received data. These instructions may include various applications used to drive each of AR display module 310, sensor module 350, imaging module 360, locating module 370, and telecommunication module 380. For example, memory 328 may store instructions used by processing component 326 to control AR display module 310 to optimize the image quality of virtual display 120.

Still referring to Fig. 3, AR display module 310 may include a micro-display 312 and an optical assembly 314. Micro-display 312 may be implemented using any technology known in the art, including, but not limited to, modulating micro-displays and emissive micro-displays. Modulating micro-displays, such as liquid crystal on silicon (LCoS), are blanket-illuminated by one or more separate light sources and modulate incident light on a pixel-by-pixel bases. In contrast, emissive micro-displays generate and emit light from the surface of the micro-displays on a pixel-by-pixel basis. The emissive micro-display may be an organic emissive micro-display, such as an organic light emitting diodes (OLED) or organic light emitting Polymers (OLEP) micro-displays. Taking OLED micro-displays as an example, OLED materials are deposited on a flat silicon backplane. Pixel circuitry may be used to convert the control signals sent by processing component 326 into current signals, which are supplied to the OLED materials via metal electrodes. In exemplary embodiments, micro-display 312 may be configured to have a size less than 0.5 inch, suitable for being installed on a wearable device. Micro-display 312 may display images in standard or high definitions. Optical assembly 314 may be used to magnify micro-display 312 so that the displayed images can be viewed by user 102.

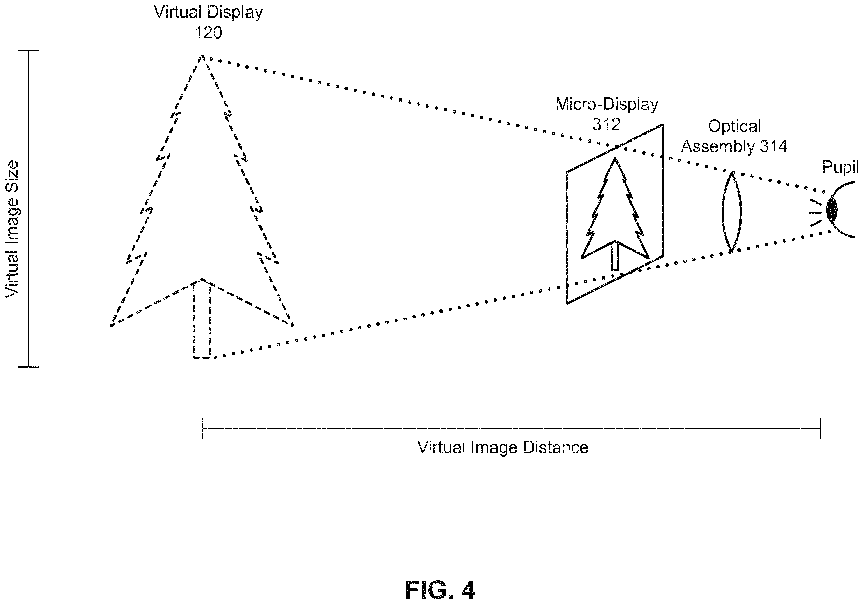

Optical assembly 314 may include any types of optical devices configured to form a magnified virtual image of micro-display 312. For example, optical assembly 314 may include a prism and a concave mirror. Also for example, optical assembly 314 may include one or more lens or lens arrays. Fig. 4 is a schematic diagram illustrating an exemplary implementation of AR display module 310. Referring to Fig. 4, optical assembly 314, placed between micro-display 312 and user 102's pupil, acts as a magnifier to produce an enlarged, virtual, and erect image of micro-display 312, i.e., virtual display 120. For example, the display area of virtual display 120 may be 100-200 times bigger than micro-display 312. With various optical designs, optical assembly 314 may be configured to form virtual display 120 at a desirable distance from the pupil and with a desirable image size, such as 4 meters and 50 inches, respectively.

In some embodiments, optical assembly 314 may also include one or more actuators configured to move the optical devices. By changing the orientations or positions of the optical devices, optical assembly 314 may adjust the distance between virtual display 120 and the pupil or the brightness of virtual display 120. This way, virtual display 120 may be properly overlaid on physical environment 160 to provide an improved experience of augmented reality.

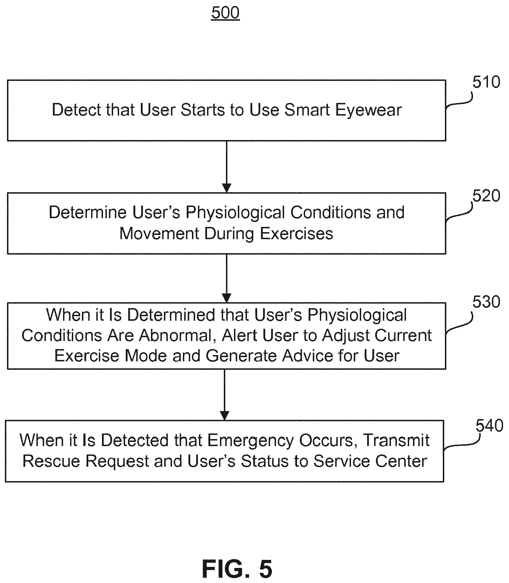

Fig. 5 is a flowchart of a method 500 according to an exemplary embodiment. For example, method 500 may be used in smart eyewear 110 depicted in Fig. 3. Referring to Fig. 5, method 500 may include the following steps 510-540.

In step 510, smart eyewear 110 detects that user 102 starts to use smart eyewear 110. For example, when sensor module 350 detects a physiological condition that is characteristic of the physiological condition of a human, controller 320 may determine user 102 starts to use smart eyewear 110. Alternatively, when sensor module 350 detects a velocity or acceleration that is typical of human movement, controller 320 may determine user 102 is wearing smart eyewear 110.

In step 520, smart eyewear 110 determines user 102's physiological conditions and movement during exercises. Controller 320 may receive, filter, analyze, process, and store the data and signals generated by sensor module 350, imaging module 360, and locating module 370. Based on these data and signals, controller 320 may determine the physiological conditions, movement, and location of user 102, and display the corresponding information on virtual display 120. If user 102 is in an outdoor environment, controller 320 may also display navigation information on virtual display 120.

In step 530, when smart eyewear 110 determines that user 102's physiological conditions are abnormal, smart eyewear 110 may alert user 102 to adjust the current exercise mode and generate an advice for user 102. Controller 320 may closely monitor user 102's physiological conditions. If one or more indexes indicative of the physiological conditions exceed a predetermined range, controller 320 may generate a warning message alerting user 102 to adjust the current exercise mode. Controller 320 may also advise user 102 to take proper actions to relieve the adverse physiological reactions. Both the warning message and the advice may be display on virtual display 120.

In step 540, when smart eyewear 110 detects that an emergency occurs, smart eyewear 110 may transmit rescue request and user 102's status to service center 150. Controller 320 may determine whether an emergency has occurred, based on the physiological conditions, movement, and position of user 102, and/or images of physical environment 160. When it is determined that an emergency has occurred, such as when user 102 has fainted, fallen down, or had a collision, controller 320 may control telecommunication module 380 to request help from service center 150. Controller 320 may also transmit the physiological conditions and position of user 102 and images of physical environment 160 to service center 150, to facilitate the diagnosis of user 102's symptom and the locating of user 102. This way, service center 150 can provide the rescue quickly.

The disclosed smart eyewear may provide several benefits. First, the smart eyewear is integrated with multiple sensors and devices to provide a comprehensive evaluation of a user's status during exercises. In particular, the smart eyewear may provide alert, advice, navigation, and rescue assistance to the user, so as to ensure the exercise safety for the user. Moreover, each of the sensors and devices may be conveniently installed and uninstalled on/from the smart eyewear. Thus, the functions of the smart eyewear may be flexibly customized based on the user's specific needs. In addition, the smart eyewear may display the exercise-related information on a virtual display and overlay the virtual display on the surrounding physical environment to create an augmented reality. This not only makes the exercises more interesting, but also greatly enriches the information received by the user.

Other embodiments of the present disclosure will be apparent to those skilled in the art from consideration of the specification and practice of the present disclosure. This application is intended to cover any variations, uses, or adaptations of the present disclosure following the general principles thereof and including such departures from the present disclosure as come within known or customary practice in the art. It is intended that the specification and examples be considered as exemplary only, with a true scope of the invention being indicated by the following claims.

It will be appreciated that the present invention is not limited to the exact constructions that are described above and illustrated in the accompanying drawings, and that various modifications and changes can be made without departing from the scope thereof. It is intended that the scope of the invention should only be limited by the appended claims.

## Claims

1. A wearable device, comprising:
a display component configured to display a virtual image;
a first sensor configured to generate a first signal indicative of a physiological condition of a user; and
a controller configured to:
determine the physiological condition based on the first signal; and
control the display component to display the physiological condition.

2. The wearable device of claim 1, wherein the controller is further configured to:
determine whether the physiological condition is within a predetermined range; and
if the physiological condition is not within the predetermined range, control the display component to display a warning message.

3. The wearable device of claim 1 or claim 2, wherein the controller is further configured to:
generate an advice based on the physiological condition; and
control the display component to display the advice.

4. The wearable device of any of the preceding claims, wherein the first sensor includes at least one of a electrocardiography (ECG) sensor, a photoplethysmogram (PPG) sensor, a galvanic skin response (GSR) sensor, a bioimpedance sensor, a heart rate sensor, or a body temperature sensor.

5. The wearable device of any of the preceding claims, wherein the display component is configured to overlay the virtual image on a physical environment surrounding the wearable device.

6. The wearable device of any of the preceding claims, wherein the display component comprises:
a display panel configured to display a first image; and
an optical assembly configured to generate a virtual image of the display panel.

7. The wearable device of claim 6, wherein the display panel is an organic light-emitting diode display.

8. The wearable device of claim 6 or claim 7, wherein the display panel and the virtual image of the display panel have different sizes.

9. The wearable device of any of the preceding claims, further comprising:
a second sensor configured to generate a second signal indicative of a movement of the wearable device,
wherein the controller is further configured to determine the movement of the wearable device based on the second signal.

10. The wearable device of claim 9, wherein the movement of the wearable device comprises at least one of an orientation, an acceleration, a velocity, a heading, or an angular rate of the wearable device.

11. The wearable device of any of the preceding claims, further comprising:
a third sensor configured to generate a third signal indicative of a location of the wearable device,
wherein the controller is further configured to determine the location of the wearable device based on the third signal.

12. The wearable device of any of the preceding claims, further comprising:
a fourth sensor configured to generate images of a physical environment surrounding the wearable device,
wherein the controller is further configured to determine a movement of the wearable device based on the images of the physical environment.

13. The wearable device of claim 12, wherein the controller is further configured to control the display component to display the images of the physical environment.

14. The wearable device of any of the preceding claims, further comprising:
a communication device configured to establish a communication with a third-party device,
wherein the controller is further configured to:
determine whether an emergency has occurred; and
if the emergency has occurred, control the communication device to send a rescue request to the third-party device.

15. A method comprising:
generating a first signal indicative of a physiological condition of a user;
determining the physiological condition based on the first signal;
displaying the physiological condition on a display panel; and
generating a virtual image of the display panel.
